Europäisches Patentamt

European Patent Office

Office européen des brevets

(11) Publication number: **0 386 441**
**A1**

## (12) EUROPEAN PATENT APPLICATION

(21) Application number: **90101679.0**

(22) Date of filing: **28.01.90**

(51) Int. Cl.5: **A61K 47/36, A61K 35/78,**
**//(A61K35/78,33:06)**

(30) Priority: **09.02.89 US 309269**

(43) Date of publication of application:
**12.09.90 Bulletin 90/37**

(84) Designated Contracting States:
**AT BE CH DE DK FR GB IT LI NL SE**

(71) Applicant: **DIAMOND SCIENTIFIC CO.**
**2538 S.E. 43rd Street**
**Des Moines Iowa 50302(US)**

(72) Inventor: **Cabrera, Francisco**
**Diamond Scientific Co., 2538 S.E. 43rd Street**
**Des Moines, Iowa 50317(US)**

(74) Representative: **Schumacher, Günter, Dr. et al**
**c/o Bayer AG Konzernverwaltung RP**
**Patentabteilung**
**D-5090 Leverkusen 1 Bayerwerk(DE)**

(54) **Calcium therapy treatment for milk fever.**

(57) A gel composition of paste-like viscosity for oral treatment of milk fever. The composition comprises in part a non-digestible water-binding swelling agent which holds and later releases in the animal's gut a source of water soluble calcium ion. The method of administration eliminates that hazards of injectables and the hazards of oral liquid solutions,

EP 0 386 441 A1

EP 0 386 441 A1

## Calcium therapy treatment for milk fever

BACKGROUND OF THE INVENTION

The present invention relates to a composition for the treatment and prevention of what is commonly known as milk fever, or more properly known as parturient paresis. This is a common metabolic disease in animals, especially in cattle, and normally occurs just prior to, but more commonly just after birthing.

Cows that have just delivered calves and are beginning lactation have an increased body demand for calcium. Because calcium is intimately involved in muscle contraction, hypocalcemia, if not corrected, may cause generalized paralysis, coma, and death. When the cows go "down", their head is thrown to the side, they are unable to move and death may result if the animal is not treated.

The common treatment for milk fever is intravenous or subcutaneous injection of an electrolyte solution of calcium borogluconate. Typically the liquid composition contains dextrose, magnesium and phosphorous salts as well. Dramatically, even within five minutes of treatment the reversal of the hypocalcemia condition begins and the animal regains muscle control and is on its feet again.

However, once an animal has gone down to milk fever, there inevitably becomes a distinct and predictable loss of milk production. Even if the animal successfully recovers, they may relapse and only get back to milk yields of 90% to 95% of the level previous to suffering of milk fever conditions.

In the past in the United States market, milk fever has been treated with intravenous and subcutaneous injections. However, these are known to cause problems. One of the problems is that the subcutaneous injection causes reactions or infection at the injection site. Intravenous injections pose the threat of cardiac arrest.

In order to overcome certain of the problems caused by subcutaneous and intravenous injections, the thought of large oral calcium solution doses has occurred. It is commonly practised in Europe. The theory being that if large, oral liquid doses are given the calcium ions may be absorbed in the digestive tract and the condition alleviated without the risk of injection. While this method of oral administration of large liquid doses achieved some success in Europe, it too has problems.

One of the most commonly occurring problems with liquid doses is that calcium chloride solutions are known to at times, cause restriction of the esophagus. Thus, it is possible for the animal to inhale the solution and have it pass into the lungs instead of the stomach. When the solution does go into the lung it commonly produces a lethal pnemonia.

There is, therefore, a real and continuing need to develop a calcium salt mixture, presented in a format which can be orally dosed to effectively treat or prevent milk fever, while at the same time eliminate the risk associated with liquid dosage. This invention has as its primary objective the fulfilling of this need.

In particular it is an objective of the present invention to provide a milk fever treating composition which is pastelike in composition so that it can be orally dosed with minimum risk of causing the esophagus restriction reaction and minimum risk of inhaling of said treatment into the lungs.

Another objective of the present invention is to provide a calcium paste composition in a dosage format which allows oral dosage and yet allows dosage in a manner in which quick absorption is achieved to provide rapid milk fever prevention or treatment.

Yet another objective of the present invention is to provide a paste composition for treating milk fever which is aqueous based, and which contains a non-digestible water-binding swelling agent which is non-harmful to the animal and which functions as a carrier for the calcium ion source such that it can be continually released for direct absorption into the animal's system, followed by animal excretion of the non-digestible swelling agent.

Another objective of the present invention is to provide a paste composition which provides a combination of a calcium ion source and a non-digestible water-binding swelling agent which provides prompt but continuous release of calcium into the animal's system to allow treatment of hypocalcemia on a fairly continuous basis such that the animal's calcium balance comes back to a proper level and remains there.

An even further objective of the present invention is to provide psyllium seed husk powder as the non-digestible water-binding swelling agent.

The method and means for achieving each of the objectives of the present invention will become apparent from the detailed description of the invention which follows hereinafter.

SUMMARY OF THE INVENTION

2

A paste composition for treating milk fever which comprises from about 0.5% by weight to about 1.6% by weight of a non-digestible water-binding swelling agent, and from 10% by weight to about 55% by weight of a water soluble calcium ion source. The non-digestible water-binding swelling agent, such as psyllium seed husk powder interacts with the water soluble calcium ion source, in the presence of an aqueous carrier, to allow prompt and continuous release from the paste of calcium directly into the animal's system. The result is effective treatment of hypocalcemia.

## DETAILED DESCRIPTION OF THE INVENTION

It is, of course, known that calcium can be used for milk fever treatment, see for example Ringharp U.S. Patent 3,461,203 issued August 12, 1969. It is also known that calcium can be used as milk fever treatment in a gel composition. However, the gel composition of the Ringharp '203 patent has not achieved success on the U.S. market, and employs a different carrying agent than the present invention, And, it is the paste composition of the present invention which does allow successful oral dosage in the back of the throat of a cow to achieve satisfactory calcium blood levels for treating milk fever without jeopardizing the animal from the standpoint of risk of lung inhalation of liquid calcium solutions.

The compositions of the present invention are a paste composition having a viscosity within the range of from about 3500 centipoises to about 15000 centipoises, preferably from about 4000 centipoises to about 10000 centipoises. The viscosity of the compositions of the present invention is inherently within these limits if the amount of each ingredient is within the range specified hereinafter,

Critical to the achievement of the effective oral administration composition of the present invention is the use of a non-digestible water-binding swelling agent for carrying on aqueous carrier based system of calcium ion source, Numerous non-digestible water-binding swelling agents have been tried but only certain ones of vegetable origin have achieved success in the present invention. In particular, the use of certain vegetable fiber seed husks provides the desirable composition. These are available for purchase on the marketplace and sold unter the trademark MuCOLIN, MuCOLIN is a non-digestible water-binding swelling agent and is believed to be Isphagula husk (dried seed coats of plantago ovata), Another commonly used name of this is psyllium seed husk.

The use Isphagula in the form of Isphagula Husk is the preferred vegetable swelling agent because of the protective properties mucopolysaccharides could offer in the gut.

Isphagula differs from other gel forming polysaccharides because:

(1) The polyxylose backbone is not broken down either by intestinal enzymes or by the intestinal flora with one exception, viz. bacteroides ovates.

(2) Isphagula contains a high amount (proportion) of galactose and residues mimicing the structure of the membrane receptors on the onterocytes.

In general, vegetable fibrous husk material which is non-digestible for cows, but which will effectively function as a water-binding swelling agent to allow prompt and continuous release of water soluble calcium ion sources can be used. The most preferred vegetable fibrous water-binding swelling agent is Isphagula Husk This is the preferred psyllium source. Psyllium hydrophilic mucilloid consists of the mucillaginous portion (the husk either milled or unmilled) of blond psyllium seeds. Psyllium hydrophilic mucilloid contains natural mucillage and forms a gelatinous mass on contact with water It is useful in the treatment of constipation by acting as a fecal softener and also as a demulcent in the presence of inflamed mucosa. The amount of non-digestible water-binding swelling agent to be used will vary within the range of from about 0.5% to about 1.6% by weight, preferably within the range of from about 1.0% to about 1.4% by weight.

The amount of calcium salt, aqueous-base carrier should range within the percentage weight of from about 10% to about 18% by weight, preferably from about 14% to 16% by weight. A source of water-soluble calcium ion can be any water soluble salt of calcium, but is preferably selected from the group consisting of chloride, phosphates, sulfates and acetate. The most preferred composition is calcium chloride in combination with dicalcium phosphate.

The balance of the system is an aqueous carrier system and can include such minors additives as magnesium hydroxide, propylene glycol, preservatives such as methyl paraben, propyl paraben, and binders such as xantham gum.

The following Table 1 illustrates the preferred compositions of the present invention on a percentage by weight basis.

Table I

| Formula Components | % Range | Preferred % Range | Best Mode % |
|---|---|---|---|
| Calcium chloride | 20-35 | 25-30 | 28.3 |
| Dicalcium phosphate | 10-20 | 13-18 | 15.3 |
| Magnesium hydroxide | 0.5-1.5 | 0.7-1.3 | 1.0 |
| Propylene glycol | 1-3 | 1.3-2 | 1.55 |
| Psyllium seed husk | 0.5-1.6 | 1-1.4 | 1.25 |
| Xantham gum | 0.3-1.4 | 0.5-0.8 | 0.62 |
| Methyl paraben | 0.016-0.020 | 0.017-0.019 | 0.018 |
| Propyl paraben | 0.018-0.022 | 0.019-0.020 | 0.020 |
| Water | Balance | Balance | |

When the milk fever treating compositions of the present invention are used, it has been found that the vegetable fiber non-digestible water-binding swelling agent interacts in some manner with the water soluble calcium ion source to provide prompt and continuous systemic responsive release of calcium to the animal. This is important because in many instances of intravenous or subcutaneous injection dosage, the animal receives an immediate upsurge in calcium level, correcting the hypocalcemia, only to abruptly show a dramatic decrease in level. The animal may then relapse into a state of paralysis again. In the present invention it has surprisingly been found that even 12 hours following administration the animal continues to have significantly higher increased levels of calcium.

While not wishing to be bound by any theory of applicability, it is believed that the invention works for several different reasons. One of those reasons is that the non-digestible water-binding fibrous swelling agent functions as a "network" for continual release of calcium into the animal's gut. Put another way, it acts as a continuous release agent, through the fiber network. Since it is non-digestible it does take time to move through the animals's system, and as it does this calcium ion is continually released. Moreover, as the material is non-digestible and highly porous, the release is abrupt and the effect continuous. In addition, the format of presentation is such that the viscosity is just right to allow the material to be administered through a dosage gun at the back of the throat without providing any significant risk of inhalation of liquid into the lungs. The esophagus restriction risk is avoided. In short, the combination of the calcium ion source and the fibrous, vegetable origin non-digestible water-binding swelling agent of this invention, preferably Isphagula Husk, produces the desirable results for successful milk fever prevention or treatment. It is believed this is so because it is a natural product based upon a non-digestible mucopolysaccharide. It is believed that this is so because it produces pastes of uniform consistency which are stable; and it is believed this is so because it has some pharmaceutical advantage as a vehicle, since there appears to be some interaction of physiologic activity between the calcium ion source and the water-binding swelling agent.

EXAMPLES

The following examples are offered to further illustrate but not limit the process of the invention.

Example 1

Procedure

The total product batch was determined to be 350 kg of which 51.9% by weight was waters. About 1/2 of the water was placed in a mixer and the calcium chloride was added, with mixing until it dissolved. The psyllium powder was added to the solution which was at a temperature above 65° C. Xantham gum was suspended in a propylene glycol solution of parabens added with mixing until it seemed dispersed.

4

Thereafter, the dicalcium phosphate and magnesium hydroxide were added with mixing. When the mixture seemed homogeneous, the remainder of the water was added and mixing continued until an appearance of homogeneous uniformity was achieved. The composition was expressed in Table 1 as "best mode".

The composition listed in Table I was tested for effectiveness in manipulation of serum calcium,

Three trials were conducted: One a 5X suggested dose levels gave almost double the calcium blood concentration in three healthy heifers from 1 hour through 4 hours, The second trial was at 5X, 2X and 1X doses in two animals and each gave a repeat of the 5X results (calcium levels were obtained at 1 hour). The 2X dose showed some absorption. The third trial was at 5X dose using the composition listed in Table 1 as "best mode". The blood samples of the animals were screened for calcium and phosphorous, and Table II below shows the results of this screening in trials one and three,

Table II

| Time/Cow# | Trial I | | | Trial III | | |
|---|---|---|---|---|---|---|
| | 601 (ctrl) | 704 | 705 | 538 (ctrl) | 541 | 481 |
| CALCIUM | | | | | | |
| 48prior | 10.1 | 9.8 | 9.7 | - | - | - |
| 24prior | 9.8 | 9.7 | 9.6 | - | - | - |
| 0 | 9.9 | 9.6 | 9.3 | 10.2 | 9.8 | 9.7 |
| 1 | 10.0 | 17.8 | 14.1 | 10.5 | 15.4 | 14.9 |
| 2 | 9.8 | 18.6 | 14.3 | 10.4 | 19.2 | 18.8 |
| 4 | 9.8 | 17.6 | 12.8 | 10.4 | 17.8 | 17.0 |
| 8 | - | - | - | 10.4 | 12.6 | 12.8 |
| PHOSPHOROUS | | | | | | |
| 48prior | 9.2 | 8.4 | 8.0 | - | - | - |
| 24prior | 9.1 | 8.4 | 7.4 | - | - | - |
| 0 | 9.4 | 7.6 | 7.2 | 8.0 | 8.2 | 7.1 |
| 1 | 9.2 | 6.4 | 7.0 | 7.7 | 8.5 | 6.5 |
| 2 | 9.6 | 8.5 | 6.7 | 7.1 | 7.8 | 7.4 |
| 4 | 9.0 | 5.9 | 6.8 | 7.4 | 7.6 | 7.3 |
| 8 | - | - | - | 7.7 | 6.5 | 5.5 |

The dosage levels suggested for the paste treating composition will vary somewhat depending upon the weight of the animal but generally should be within the range of about 0.5 grams per kg of body weight to about 1.5 grams per kg of body weight, preferably from 0.6 to 1.3.

The product, as earlier expressed, is a suitable paste composition of correct viscosity for packaging in a non-harmful, nontoxic tube. This can then be put in a dosage gun, very similar to a caulking gun and inserted into the animal's throat, way to the back, and dosed. The typical dosage used in the trials was 355 grams of the composition.

These compositions have been shown to be stable for up to several months after preparation when sealed in inert polymer plastic tubes. The use of the non-digestible water-binding swelling agent is found to pro vide at least the following advantages: a controllable paste viscosity; a stable paste that does not separate, harden, or lose it viscosity with time; a stable paste that is compatible with the essential salts and adjunct components of the formula; a stable paste of relatively low irritation compared to a salt solution; a stable paste with a low taste profile compared to the salt solution; and a safer route of administration.

**Claims**

1. A paste composition for treating milk fever, comprising:
   (a) from about 0.5% to about 1.6% by weight of a non-digestible water-binding swelling agent;
   (b) from about 10% to about 55% by weight of a water soluble calcium ion source; and
   (c) an aqueous carrier.

2. The paste composition of claim 1 which has a viscosity within the range of from 3500 cp to 15000 cp.

3. The past composition of claim 1 which is encapsulated in an inert discharge tube,

4. The paste composition of claim 1 wherein the water soluble calcium ion source is salts of calcium selected from the group consisting of chloride, phosphates, sulfates, and acetate.

5. The paste composition of claim 4 wherein the water soluble calcium ion source is selected from the group consisting of chloride and phosphates.

6. The composition of claim 1 wherein said non-digestible water-binding swelling agent is a vegetable fiber material,

7. The composition of claim 6 wherein said vegetable material is Isphagula Husk.

8. The composition of claim 7 wherein the amount of Isphagula Husk is from about 1.0% by weight of said composition to about 1.4% by weight of said composition.

9. A method of treating milk fever in cows, comprising:

(a) preparing a paste composition which comprises from about 0.5% to about 1.6% by weight of a fibrous vegetable, non-digestible water-binding swelling agent, and from about 10% to about 55% by weight of a water soluble calcium ion source with the balance being an aqueous carrier; and thereafter

(b) administering said paste composition orally to a cow suffering from hypocalcemia.

European Patent Office

**PARTIAL EUROPEAN SEARCH REPORT**
which under Rule 45 of the European Patent Convention
shall be considered, for the purposes of subsequent
proceedings, as the European search report

Application number

EP 90 10 1679

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | PHARM. ACTA HELV., vol. 52, no. 9, 1977, pages 214-217; A.G. MATTHA: "Rheological studies on plantago albicans (psyllium) seed gum dispersions. II. Effect of some pharmaceutical additives" <br><br> * Page 215, "2. Effect of electrolytes" <br><br> -- | 1-8 | A 61 K 47/36 <br> A 61 K 35/78 <br> (A 61 K 35/78, <br> A 61 K 33:06) |
| Y | DE-A-2 505 755 (F. SAGMEISTER et al.) <br><br> * Claims 1-3,7 * <br><br> -- | 1-8 | |
| Y,D | US-A-3 461 203 (N.E. RINGARP) <br><br> * Column 3, lines 6-10; column 3, lines 47-53 * <br><br> ---- | 1-8 | |

TECHNICAL FIELDS
SEARCHED (Int. Cl.4)

A 61 K

### INCOMPLETE SEARCH

The Search Division considers that the present European patent application does not comply with the provisions of the European Patent Convention to such an extent that it is not possible to carry out a meaningful search into the state of the art on the basis of some of the claims.
Claims searched completely: 1-8
Claims searched incompletely:
Claims not searched: 9
Reason for the limitation of the search:

Method for treatment of the human or animal body by surgery or therapy (see Art. 52(4) of the European Patent Convention).

| Place of search | Date of completion of the search | Examiner |
|---|---|---|
| THE HAGUE | 15-05-1990 | BERTOCHHI |